# EUROPEAN PATENT SPECIFICATION

(11) **EP 2 683 337 B1**
(45) Date of publication and mention of the grant of the patent: **16.08.2017**
(21) Application number: 11709284.1
(22) Date of filing: 10.03.2011
(51) Int. Cl.: A61F 2/28, A61F 2/30, A61F 2/44, A61F 2/46, A61B 17/16, A61B 17/80, A61B 17/86

(54) **AWL SCREW FIXATION MEMBERS AND RELATED SYSTEMS**
AHLENSCHRAUBSICHERUNGSELEMENTEN UND ENTSPRECHENDE SYSTEME
ÉLÉMENTS DE FIXATION À VIS ALLEN ET SYSTÈMES ASSOCIÉS

(43) Date of publication of application: 15.01.2014
(73) Proprietor: Synthes GmbH, 4436 Oberdorf (CH)
(72) Inventor: SCHOENLY, Jared, West Chester, PA 19380 (US); EVANS, David, West Chester, PA 19380 (US); FENN, Matthew, West Chester, PA 19380 (US)
(74) Representative: Atkinson, Ian Anthony
(86) International application number: PCT/US2011/027849
(87) International publication number: WO 2012/121726

(56) References cited:
- EP-A1- 1 787 591
- WO-A1-00/66011
- WO-A1-2010/089481
- DE-A1-102009 033 138
- DE-U1- 20 317 651
- GB-A- 2 458 935
- US-A1- 2008 294 260
- US-A1- 2008 300 634

## Description

### BACKGROUND

Bone screws are commonly used to fix adjacent bones or bone fragments with respect to each other, or to attach structure to bone. For example, bone screws are commonly used to help repair fractures in bone, to attach bone plates to bone, to fix adjacent vertebral bodies, and the like.

Existing bone screws and conventional methods of bone screw insertion can, however, introduce undesirable complications in such procedures. For example, conventional methods of bone screw insertion can lead to, *inter alia,* small and/or mobile bone fragments dislocating from the bone or bone segment due to axial pressure and insertion torque transmission during screw insertion; screw loss during operation (including transporting the screw from its storage place to final fixation location in the patient); shear off and cam out of the screw head during screw insertion and/or removal; slipping between the screw driver interface and the screw driver; stripping of the screw driver interface; bone milling during rotational insertion of self drilling and/or self tapping screws; misalignment of the pre-drilled holes in adjacent bone fragments and/or bone plates which can lead to secondary dislocation and inaccurate positioning of the bone fragments and/or bone plate; suboptimal screw fixation due to angular misalignment of a pre-drilled pilot hole's axis and the desirable screw insertion axis; and post operative back-out of screws.

In some cases, when conventional bone screws are used to attach small bone segments that have little structural support, the axial and rotational force required to start a screw into such small fragments can be such that the fragment becomes dislocated. Additionally, when it is desirable to use a long bone screw, driving the screw into bone can be laborious and time consuming.

Existing bone screw fixation systems also, in some cases, require the user to form a pilot hole in the bone so as to provide a hole with which the screw threads can engage. Forming this pilot hole, however, is labor-intensive and time-consuming, and can complicate the fixation procedure.

German Patent Publication No. DE-102009033138 discloses a screw configured to compress bone. The screw includes a head threaded portion, a distal threaded portion, and a non-threaded shank portion separating the head threaded portion and the distal threaded portion. A conical attachment tip is formed at an end of the distal threaded portion.

International Patent Publication No.WO-2010/089481 discloses a screw configured to stabilize and compress bones following orthopedic surgery on the hand or foot. The screw comprises a barrel that includes barrel portions which correspond to distal and proximal portions of the screw, respectively. The distal and proximal portions of the screw include threads as well as teeth. The barrel has an unthreaded portion which extends from the distal barrel portion to the proximal barrel portion.

### SUMMARY

The present invention provides a bone implant assembly as claimed in claim 1.

Optional further features of the bone implant assembly are defined in the dependent claims.

The present disclosure provides bone implant assemblies, the assemblies including an implant that may include opposed bone-engaging surfaces and further defines at least one aperture extending therethrough; and a bone anchor configured for insertion into the aperture so as to extend through the at least one aperture and into a bone, thereby affixing the implant to the bone, the bone anchor including: a proximal end, a distal end opposite the proximal end, and an intermediate portion extending therebetween. The distal end defines a tip configured to cut into the bone. At least a portion of the shaft is unthreaded from the tip and along a portion of the intermediate portion toward the proximal end. The proximal end of the bone anchor may define an exterior thread configured to engage a complementary thread of the implant in the aperture.

Also disclosed herein are bone anchors including a body comprising a tip, a shaft that extends proximally from the tip, and an externally threaded head extending proximally from the shaft, the tip configured to penetrate into a bone, wherein at least a portion of the shaft is unthreaded and extends proximally from the tip; and an engagement feature configured to engage a complementary feature of a driving instrument that is configured to apply a torsional force to the bone anchor so as to drive the tip into the bone.

### DESCRIPTION OF THE DRAWINGS

The foregoing summary, as well as the following detailed description of the preferred embodiments of the application, will be better understood when read in conjunction with the appended drawings. For the purposes of illustrating the present disclosure, there are shown in the drawings preferred embodiments. It should be understood, however, that the instant application is not limited to the precise arrangements and/or instrumentalities illustrated in the drawings, in which:
Fig. 1A is a perspective view of a bone anchor constructed in accordance with one embodiment;
Fig. 1B is a side elevation view of the bone anchor illustrated in Fig. 1A;
Fig. 1C is a front elevation view of the bone anchor illustrated in Fig. 1A;
Fig. 2A is a perspective view of a bone anchor constructed in accordance with another embodiment;
Fig. 2B is a side elevation view of the bone anchor illustrated in Fig. 2A;
Fig. 2C is a front elevation view of the bone anchor illustrated in Fig. 2A;
Fig. 3A is a perspective view of a bone anchor constructed in accordance with another embodiment;
Fig. 3B is a side elevation view of the bone anchor illustrated in Fig. 3A;
Fig. 3C is a front elevation view of the bone anchor illustrated in Fig. 3A;
Fig. 4A is a perspective view of a bone anchor constructed in accordance with another embodiment;
Fig. 4B is a side elevation view of the bone anchor illustrated in Fig. 4A;
Fig. 4C is a front elevation view of the bone anchor illustrated in Fig. 4A;
Fig. 5A is a perspective view of a bone anchor constructed in accordance with another embodiment;
Fig. 5B is a side elevation view of the bone anchor illustrated in Fig. 5A;
Fig. 5C is a front elevation view of the bone anchor illustrated in Fig. 5A;
Fig. 6A is a perspective view of a bone anchor constructed in accordance with another embodiment;
Fig. 6B is a side elevation view of the bone anchor illustrated in Fig. 6A;
Fig. 6C is a front elevation view of the bone anchor illustrated in Fig. 6A;
Fig. 6D is a side elevation view of the bone anchor similar to Fig. 6B, but including threads constructed in accordance with another embodiment;
Fig. 7 is a perspective view of a bone implant assembly in accordance with one embodiment, including an implant and a plurality of bone anchors;
Fig. 8 is an exploded perspective view of the bone implant assembly illustrated in Fig. 7;
Fig. 9 is a side elevation of the bone implant assembly illustrated in of Fig. 7;
Fig. 10 is an exploded view of a bone implant assembly constructed in accordance with another embodiment;
Fig. 11 is an exploded view of the bone implant assembly illustrated in Fig. 10;
Fig. 12 is an exploded view of a portion of the bone implant assembly illustrated in Fig. 10;
Fig. 13A is a top plan view of a fixation plate of the implant illustrated in Fig. 10;
Fig. 13B is a front elevation view of the fixation plate illustrated in Fig. 10A;
Fig. 13C is a top elevation view of fixation plate similar to the fixation plate illustrated in Fig. 13A, but constructed in accordance with another embodiment;
Fig. 13D is a front elevation view of the fixation plate illustrated in Fig. 10C;
Fig. 14A is a side elevation view of a bone anchor constructed in accordance with another embodiment;
Fig. 14B is a side elevation view of a bone implant assembly including an implant and a bone anchor, showing the bone anchor inserted through an implant and partially inserted into a pilot hole of an underlying bone;
Fig. 14C is a side elevation view of the bone implant assembly illustrated in Fig. 14B, showing the bone anchor further driven into the pilot hole such that threads of the bone anchor engage the underlying bone;
Fig. 14D is a side elevation view of the bone implant assembly illustrated in Fig. 14C, showing the bone anchor further driven into the bone and seated against the implant;
Fig. 15A is an exploded view of an exemplary bone implant assembly;
Fig. 15B is a side elevation view of the bone implant assembly of Fig. 15A, with the fixation plate and spacer assembled together;
Fig. 15C is a perspective view of the bone implant assembly of Fig. 15A, with the fixation plate and spacer assembled together;
Fig. 15D is a top elevation view of the bone implant assembly of Fig. 15C; and
Fig. 15E is a rear elevation view of the bone implant assembly of Fig. 15D.

### DETAILED DESCRIPTION

The present disclosure may be understood more readily by reference to the following detailed description taken in connection with the accompanying Figs. and examples, which form a part of this disclosure. It is to be understood that this disclosure is not limited to the specific devices, methods, applications, conditions or parameters described and/or shown herein, and that the terminology used herein is for the purpose of describing particular embodiments by way of example only and is not intended to be limiting of the present disclosure.

Also, as used in the specification including the appended claims, the singular forms "a," "an," and "the" include the plural, and reference to a particular numerical value includes at least that particular value, unless the context clearly dictates otherwise. The term "plurality", as used herein, means more than one. When a range of values is expressed, another embodiment includes from the one particular value and/or to the other particular value. Similarly, when values are expressed as approximations, by use of the antecedent "about," it will be understood that the particular value forms another embodiment. All ranges are inclusive and combinable.

It is to be appreciated that certain features of various embodiments set forth in the present disclosure which are, for clarity, described herein in the context of separate embodiments, may also be provided in combination in a single embodiment. Conversely, various features of the present disclosure that are, for brevity, described in the context of a single embodiment, may also be provided separately or in any subcombination. Further, reference to values stated in ranges includes each and every value within that range.

Certain terminology is used in the following description for convenience only and is not limiting. The words "right", "left", "top" and "bottom" designate directions in the drawings to which reference is made. The words "inwardly" and "outwardly" refer to directions toward and away from, respectively, the geometric center of the device and designated parts thereof. The words, "anterior", "posterior", "superior", "inferior", "lateral", "medial", "sagittal", "axial", "coronal," "cranial," "caudal" and related words and/or phrases designate preferred positions and orientations in the human body to which reference is made and are not meant to be limiting. The terms "anchor" and "fixation member" may be used interchangeably.

The disclosed components will now be described by way of reference to the appended figures.

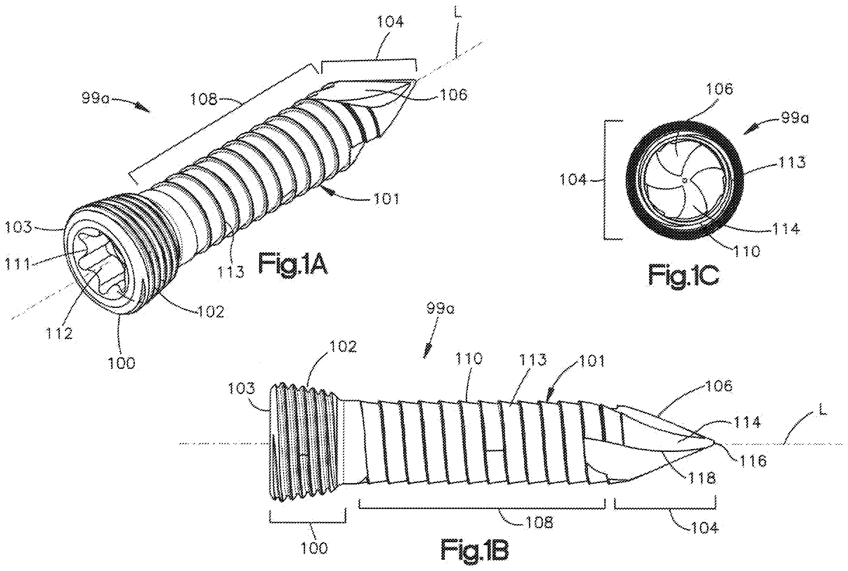

Referring now to Figs. 1A-1C, a bone fixation member, such as bone anchor 99a, includes a body 101 that is elongate along a central longitudinal axis L, and defines a proximal end 100, a distal end 104 spaced from the proximal end 100 along the longitudinal axis L, and disposed opposite the proximal end 100, and an intermediate portion 108 disposed between the proximal end 100 and the distal end 104.

The bone anchor 99a defines a tip 116 at the distal end 104 that is capable of penetrating or cutting a vertebral body (e.g., bone) or other structure. The proximal end 100 of the bone anchor 99a defines a head 103 that is suitably configured so as to engage at least one complementary engagement feature 111 of a driving instrument, such as a screwdriver or other driver device, that applies a force that biases the tip of bone anchor 99a into an underlying bone, such as a vertebral body.

The bone anchor 99a can further include a shaft 113 that can have a substantially constant diameter and extends between the head 103 and the tip 116. The shaft can also increase in diameter along a direction from the tip 116 toward the head 103, however the slope of the outer surface of the shaft 113 can be different from that of the tip 116.

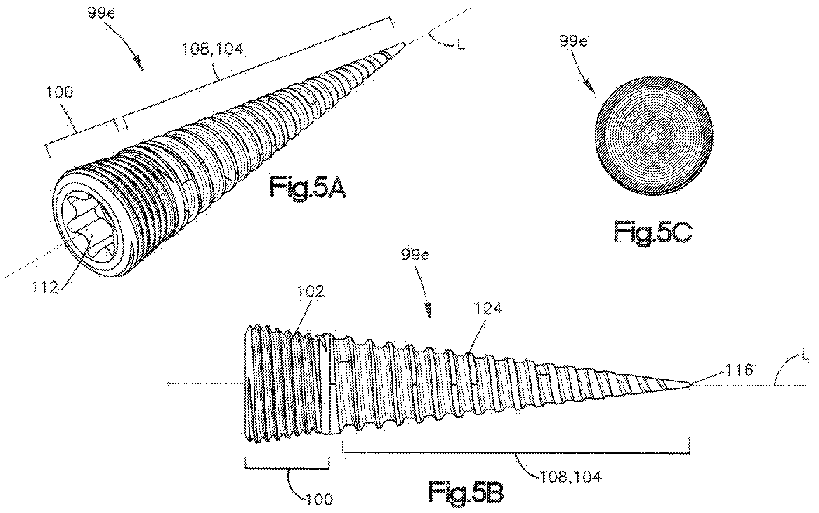

The shaft 113 may have a diameter that remains essentially constant over its length. Alternatively, for instance as shown in Fig. 5A, the diameter of the shaft 113 may vary, for instance, along a direction from the distal end to the proximal end by any amount as desired. For instance, the outer surface of the shaft 113 can define an angle of from about 3 degrees to about 15 degrees. Accordingly, the diameter of the distal end of the shaft can be reduced, in some embodiments, relative to the diameter of the proximal end of the shaft 113 by from about 99.65% to about 99.8%. The shaft 113 may be of essentially constant diameter along a portion of its length, and include a region of varying diameter.

The distal end 104 can have a longitudinal length relative to that of the shaft 113 as desired. For instance, the length of the taper on distal end 104 to the total length of shaft below the screw head (108+104)) can be from about 1:2 to about 1:5.

The length of the unthreaded portion of the shaft 113 may be from about 10 mm to about 25 mm, or from about 12 mm to about 20 mm. The ratio of the length of the threaded portion of the shaft 113 to the length of the anchor below the head (i.e., 104 + 108) is suitably in the range of from about 1:1 to 1:10, or from 1:2 to about 1:5. The radial height of the threads 126 on the threaded portion of the shaft can be from about 0.1 mm to about 0.5 mm, or even from about 0.2 mm to about 0.3 mm. Adjacent threads 126 may be spaced from one another by from about 0.8 mm to about 2 mm, or even by from about 0.9 mm to about 1.8 mm.

In this regard, it should be appreciated that when the shaft 113 defines a substantially constant diameter, the outer surface of shaft 113 also defines a slope different from that of the tip 116. It should be further appreciated that the slope of the outer surface of the shaft 113 can be substantially equal to that of the tip 116 (see Figs. 5A-C). The engagement member 111 of the head 103 can be provided as a recess 112 that is star-shaped as illustrated, but can be cross-shaped, pyramidal, hexagonal, helical, or other configurations known in the art that facilitate robust engagement between the anchor and the driving instrument. The StarDrive™ system from Synthes (www.synthes.com) is considered a suitable system for driving the anchors described herein. In some embodiments (not shown), the bone anchor 99a defines a tip 116 and shaft 113 that extends proximally from the tip 116. The shaft may have a proximal end (at a distance from the tip 116), which end is adapted to engage with a driving instrument. For example, the proximal end may include a recess as described above.

The engagement member 111 can alternatively be configured as a protrusion as desired that is configured so as to engage with a driver device that applies a distal biasing force to the bone anchor 99a so as to implant the bone anchor 99a into the underlying bone. Such a protrusion may have a cross-section that is triangular, square, pentagonal, hexagonal, or otherwise shaped as desired. The protrusion can, for instance, be received by a socket or other grip of the installation device.

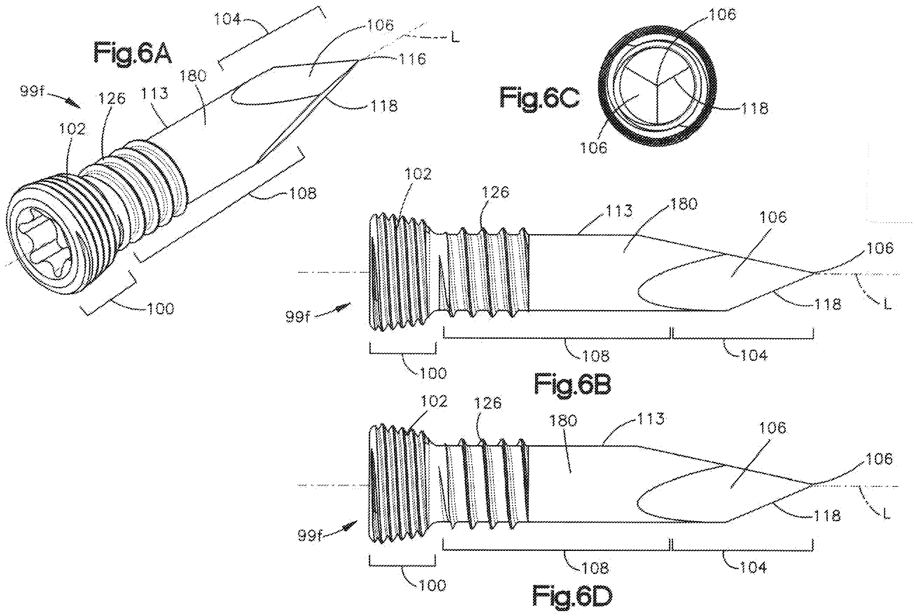

The bone anchor 99a can be configured as a bone screw, whereby the intermediate section 108 of the bone anchor body 101 defines an exterior feature, such as a thread 110 that extends about the shaft 113. The exterior feature can be configured as a bone thread that is adapted to securably engage with the underlying bone into which the bone anchor is implanted. The thread 110 may be helical in configuration (e.g., helical thread 126 in Fig. 6B), or may be a stepped, helical thread in configuration, as illustrated in Figs. 1A-B. The thread 110 may be pyramidal in cross-section and have a sharp distal end.

The thread 110 may alternatively define a flat distal end, a rounded distal end, or any alternatively sized or shaped distal end as desired. The thread 110 spans only a portion of the intermediate region 108, as shown by the thread 126 in Fig. 6B. The user can drive the bone anchor 99a into the underlying bone until the thread 110 contacts the bone, at which point the user may then apply a torsional force to the bone anchor 99a so as to screw the bone anchor 99a into the bone for final seating.

The thread 110 may have the cross-section of an obtuse or scalene triangle, which cross-section allows the installed fixation body to resist a pull-out force. Thread 110 allow bone anchor to be installed by applying of a torsional force to the bone anchor 99a so as to advance the thread 110 into the underlying bone. The thread 110 defines a height that extends out from the shaft 113 along a direction angularly offset from the longitudinal axis L of the bone anchor 99a, such as substantially perpendicular to the longitudinal axis L. The height can be substantially constant, or can vary along the length (e.g., along longitudinal axis L) of the shaft 113. For example, the thread 110 may have a height that is larger (i.e., is taller) closer to the proximal end 100 of the anchor 99a and that is smaller (i.e., shorter) closer to the distal end 104 of the anchor 99a. In one exemplary embodiment, the thread 110 at the distal end of the anchor 99a may have a height of x, and the thread 110 at the proximal end of the anchor may have a height of 1.3x. Alternatively, the thread 110 may have a height that is constant along the length of the bone anchor. In one such embodiment, the thread 110 may have a height of x at all thread locations. In yet another embodiment (not shown), the intermediate region 108 of the anchor 99a tapers from the proximal to the distal ends of the anchor 99a, but the thread 100 varies in height along the longitudinal axis L such that that the diameter of the anchor 99a is constant.

The bone anchor 99a can further define an external thread 102 located at the proximal end 100 of the body 101, for instance at the head 103. The external thread 102 is suitably configured to engage a complementary thread in another component, such as an implant, so as to provide locking fixation between the implant and the underlying bone. For example, the external thread 102 may engage an internal thread of an aperture in a fixation plate or other device into which the anchor is installed. The Synfix™ system from Synthes (www.synthes.com) is one example of such a locking system.

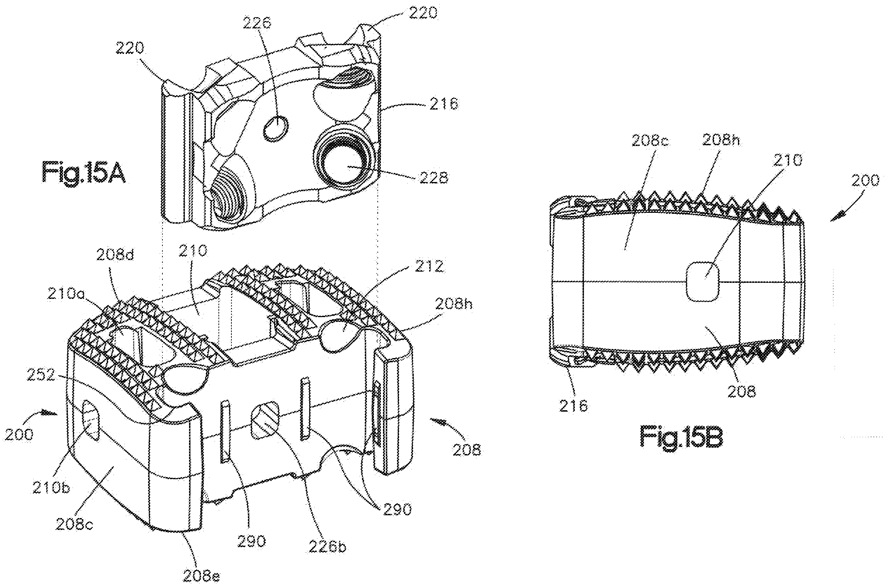

In some embodiments, the aperture may be an enclosed channel extending through a portion of the implant. Such an embodiment is shown by figure 15a, in which aperture 228 extends through the fixation plate 216. Channels that are circular in cross-section are considered suitable apertures. In some embodiments, the channel is fully enclosed within the implant. The channel, however, need not be fully enclosed within the implant; in some embodiments, the aperture may be a channel or slot that is at least partially open to the environment exterior to the implant.

The exterior thread 102 of the bone anchor may be a dual lead thread; such dual leads enable the user to more quickly implant the bone anchor into a component that bears a complementary thread. The external thread 102 may be an external helical thread. In some embodiments, the proximal end of the bone anchor 99a includes one or more splines that engage with complementary structures in a fixation body or other component.

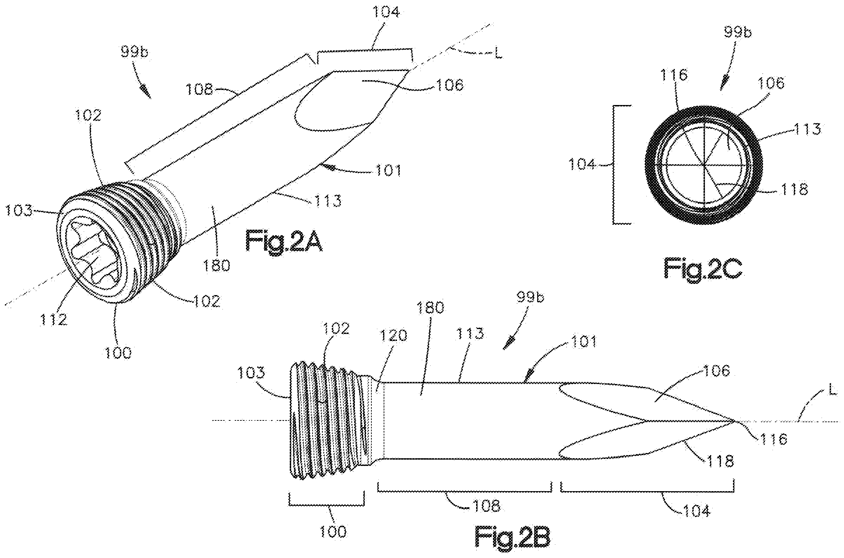

The tip 116 of the bone anchor 99a is configured so as to penetrate or cut vertebral bone so as to enable secure insertion of the bone anchor into the vertebral body. As shown in Fig. 1, the distal end 104 is adapted to penetrate bone. The distal end 104 includes a sharp tip 116, and can further include cutting facets 114 that can extend helically about the tip 116. By reference to Fig. 2, the distal end 104 is configured as a trocar tip. Such tips allow the user to implant the bone anchor (at least partially) into the vertebral body by hammering or otherwise forcing the tip into the vertebral body; a pilot hole is not always needed. Trocar tips may be pyramidal or multi-faceted; the distal end 104 shown in Figure 2B is pyramidal in configuration.

An awling motion or other back-and-forth reciprocating motion may be used to effect penetration of the tip 116 into the underlying bone, which awling motion in turn biases the tip 116 against the bone and effects cutting or penetration. The awling may be a twisting/torquing back-and-forth motion while the anchor 99a is biased into the underlying bone. This may be contrasted with a screwing-type motion in which the anchor 99a is rotated in a single direction while being biased or otherwise driven into the underlying bone.

In one non-limiting example, the user may engage a screwdriver or similar implement into recess 112 of the anchor 99a, and then apply an awling motion to the anchor 99a so as to install the anchor into underlying bone. In some embodiments, the user may form a pilot hole in the underlying bone. Such pilot holes, however, are not necessary, and the anchor 99a may be configured so as to permit installation into underlying bone without the use of a pilot hole. In other cases, the tip 116 is driven distally into the underlying bone and penetrates into the bone in a nail-like manner.

In other embodiments, the tip 116 is driven into the underlying bone, and the anchor 99a is further inserted into the bone by way of the described awling motion. In other embodiments, the tip 116 is driven (e.g., via hammering) into the underlying bone, and the anchor 99a is itself then further driven into the bone by way of a hammering or nailing force. Anchors 99a may thus be installed by a nailing or hammering force, an awling, or some combination. The anchor 99a may also be configured - e.g., with a helical thread - so as to be installed by application of a screwing force. It is to be understood that the above-described techniques are applicable to any of the anchors 99a, 99b, 99c, 99d, 99e, 99f, and 99g disclosed in Figures 1-6, 14, and elsewhere herein, and that the various described anchors do not limit the scope of this disclosure.

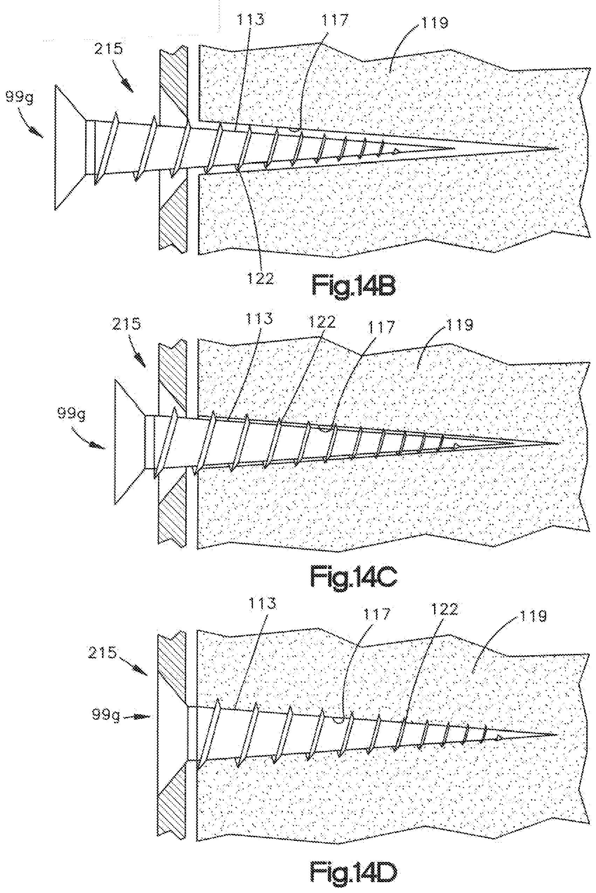

The anchor may be constructed such that the tip 116, the distal end 104, or both, may be installed by a nailing, hammering, or awling motion, and the remainder of the anchor 99a is then installed by a screwing motion. The tip 116 of the bone anchor 99a is configured as a trocar tip that can include multiple facets 106, that are separated from one another by sharp edges 118, and thus configured to drive into the underlying bone. The tip 116 may have a helical or screw-like configuration, as shown by the distal end 104 in Fig. 1B. The anchor 99a can thus increase the speed of implantation as compared to existing bone screws that receive a continuous torsional driving force. The bone anchor 99a can be implanted into a pre-formed pilot hole that extends into the underlying bone (see, for instance Figs. 14B-D).

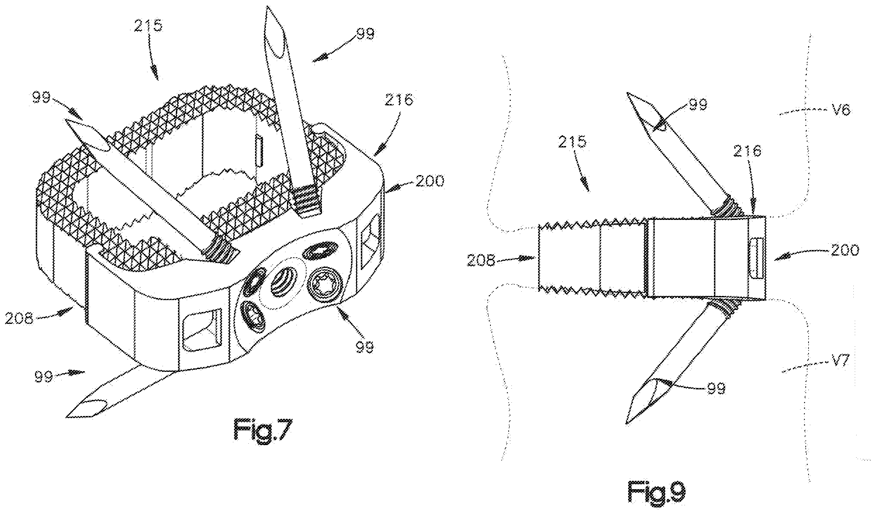

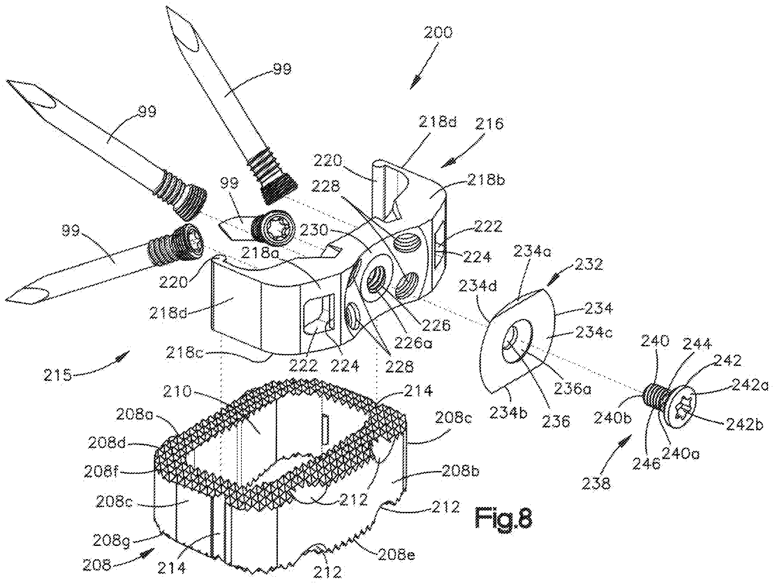

With continuing reference to Figs. 1A-C, the bone anchor 99a can further include an external thread 102 at the proximal end 100, for instance at the head 103, the thread 102 configured to engage with a complementary thread of a bone implant, such as a bone fixation plate. The head 103 can further be conical in shape, and is configured to be used in conjunction with a receiving aperture in another component, such as an implant, which receiving aperture is cylindrical or even conical in configuration. Accordingly, the depth of penetration of the bone anchor 99a can be controlled, as the head 103 cannot be advanced beyond the point at which the conical head has fully engaged with the conical receiving aperture (see e.g., Figs. 7-8).

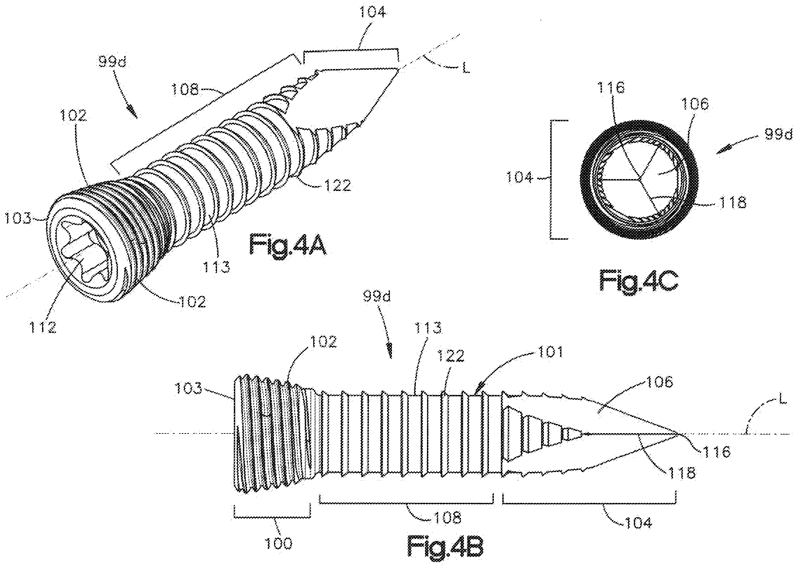

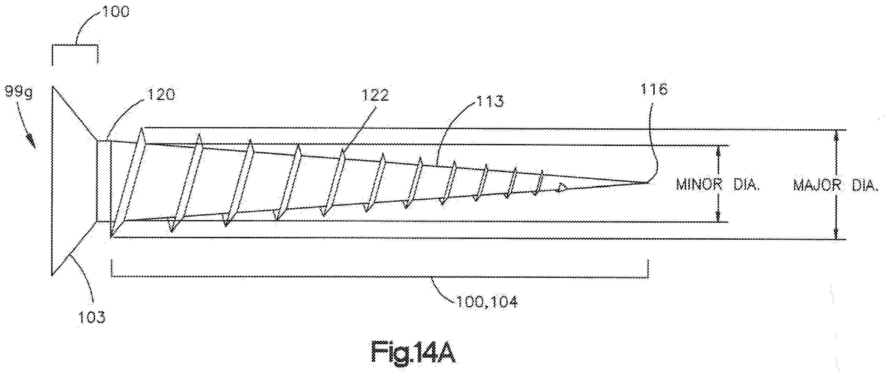

Referring now to Figs. 4A-C, the exterior feature of the intermediate section 108 can include ridges 122. The ridges 122 may all be of the same height or of different heights. The ridges 122 may be of the same height along the entirety of the intermediate section 108, or of differing heights along the entirety of the intermediate section. In one embodiment, the ridges 122 define a greater height at the proximal end of the anchor than at the distal end of the anchor, as shown in, e.g., Figure 14A. In accordance with the illustrated embodiment, the ridges 122 encircle at least a portion of the intermediate portion 108. The ridges 122 may be configured so as to resist proximally-directed pull-out forces when the bone anchor 99a has been installed into a body, such as underlying bone.

The ridges 122 also allow the bone anchor to be implanted by application of a distal driving force, such as a hammering applied to the head 103. This force may be applied by way of a mallet or by mechanical means, such as a sonic hammer or other driver. A pinion drive may also be used to drive the anchor into the vertebral body. The ridges may, as described elsewhere herein, be characterized as being a right triangle in cross-section. The ridges may also be scalene, equilateral, or obtuse triangles in cross-section. The ridges may all be of the same height; some of the ridges may be of different heights from one another. Furthermore, the tip 116 can be pointed, and thus devoid of cutting facets 114 illustrated in Figs. 1A-1C.

Ridges 122 are suitably triangular in cross-section so as to resist pull-out when the anchor has been inserted into vertebral bone or other material. While the exemplary embodiment shown in Fig. 4 includes a plurality of ridges 122 that can be concentric, and can be arranged along essentially the entire length of the intermediate region 108, the ridges 122 of any of the bone anchors described above can extend along all or a portion of the length of the intermediate region 108, such as the shaft 113. For example, the ridges may be present only at the part of the intermediate region that is immediate adjacent to the proximal end 100. Alternatively, the ridges may be present at the part of the intermediate region that is adjacent to the distal end 104 of the anchor. The anchor may bear one, two, three, or more ridges. The ridges may configured such that the intermediate region includes one or more "ridge-less" regions that are smooth and free of ridges. One such region is shown by unthreaded region 180 in Figure 2A.

The distal end 104 of the bone anchor embodiment 99d of Figs. 4A-C features a pyramidal tip 116 that defines a plurality of facets 106 separated from one another by longitudinally extending edges 118. In this particular embodiment, the ridges 122 are present on portions of the distal end 104 that are not facets of the tip.

An alternative variant is shown in Figs. 2A-C, showing that the intermediate region 108 of the bone anchor 99b may be substantially smooth and thus free of ridges, screw threads, and the like. The intermediate region 108 may be of essentially constant cross-section (as shown), but can also taper inwardly toward the longitudinal axis L along a direction from the proximal end 100 toward the distal end 104 of the bone anchor. The bone anchor 99b may include a neck 120 or other transition region disposed between the head 103 and the shaft 113 of the bone anchor 99b.

The neck 120 may act to prevent over-insertion of the anchor into an implant device, so as to control the depth to which the anchor is inserted. As one example, when the anchor shown in Figs. 2A-C is inserted into a bone implant (e.g., a bone fixation plate), the thread 102 of the proximal end 100 engages with a complementary thread of the implant. Once the exterior thread 102 of the anchor is fully engaged with the complementary thread of the implant, the collar 120 of the anchor can contact a flange of the implant. The flange can be sized so as to prevent passage of the collar 120. In this way, the inventive anchors and systems may be configured to control the depth of anchor penetration.

In the case of the variant bone anchor 99b shown in Fig. 2A-C, the anchor may be installed by biasing (*e.g.,* by hammering) the tip of the anchor into the bone material of interest. The user may then further insert the anchor into the bone by application of additional force (by hammering or by applying a constant pushing force, such as force applied by a pinion or screw drive). Once the anchor has been sufficiently inserted into the subject that the locking means (e.g., the external thread 102 of the proximal end 100) engages with a complementary thread on another component of an implant, such as a fixation plate.

The user may then apply a twisting force via the recess 112 in the anchor so as to fully engage the thread 102 of the anchor and to lock the anchor into place. Such anchors enable robust, rapid insertion into subjects, as twisting force need be applied only at the end of the procedure in order to lock the anchor into place. Fig. 2C illustrates a front elevation of the anchor. In an alternative embodiment (not shown), the anchor 99b may include a ridge or other structure disposed at the proximal end 100 that securably engages with a complementary feature on another component of an implant, such as a fixation plate. In one non-limiting embodiment, the aforedescribed ridge engages with a complementary ring within a socket of another component of an implant, the complementary ring being sized such that some force is required to advance the ridge beyond the ring. Once so advanced, the ridge and anchor are held in place by the ring.

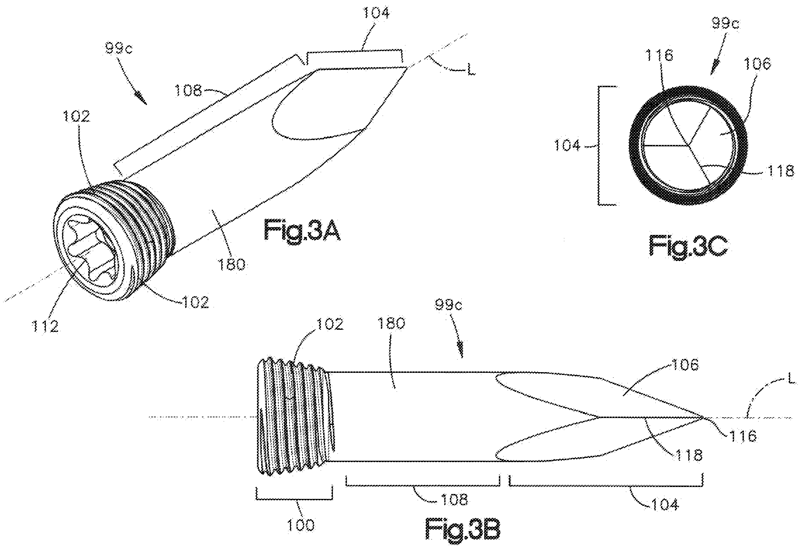

Referring to Figs. 3A-3C, the bone anchor 99c is constructed substantially as illustrated in Figs. 2A-C, but is devoid of a collar or other transition region. The anchor of Figs. 3A-3C is thus locked into place by engagement of the conical head 100 and associated thread 102 with a complementarily-shaped conical socket and thread of the implant component into which the anchor is inserted. The other numbered elements of Figs. 3A-3C are explained by reference to the like-numbered elements of Figs. 2A-2C. As shown by exemplary, non-limiting figure 3A, the anchor 99c includes an unthreaded region 180, which region may be smooth (as shown), or may include ridges, spikes, or teeth (not shown).

Referring now to Figs. 5A-C, the illustrative bone anchor 99e the intermediate section 108 is tapered inwardly along a longitudinal direction (illustrated by longitudinal axis L) from the proximal end 100 toward tip 116. As shown by the side elevation of Fig. 5B, the anchor's proximal end 100 includes an external thread 102, which thread is suitably configured to engage with a complementary thread on an implant component so as to lock the inserted anchor into place. The anchor includes a recess 112 for engaging a delivery device, such as a screwdriver or mechanized device. The anchor may alternatively include a protrusion extending from the proximal end of the anchor, which protrusion may engage with a delivery device so as to allow a user to apply a bias to the anchor. As one example, the protrusion may be hexagonal in cross-section so as to mate with a complementary hexagonal recess of a delivery device.

The bone anchor further includes a thread 124 that extends along the intermediate region and distal end 108 and 104. In accordance with the illustrated embodiment,, the thread 124 is of variable height (or even of variable pitch), and runs from a lower (shorter) height at the distal end 104 to a higher (taller) height closer to the proximal end 100 of the bone anchor 99e. This configuration enables the user to seat the bone anchor 99e into an existing pilot hole formed in bone (e.g., by awling or by operation of a drill or other suitable instrument). By applying a twisting force to the bone anchor 99e seated in the pilot hole, the user can seat all of the threads of the anchor within the bone by using fewer turns than would be needed to seat every thread of the anchor if the anchor had to penetrate the bone starting with its tip. In an alternative embodiment (not shown), the anchor 99e shown in Figure 5A includes ridges (now shown) in place of thread 124. Such a ridged anchor may then be driven (via, e.g., hammering or awling) into underlying bone.

Referring to Figs. 6A-6D, the illustrative bone anchor 99f includes an external thread 126 that extends along a portion of the intermediate region 108 (as shown in Fig. 6B). The bone thread 126 may be triangular in cross-section, or may alternatively have a flattened or rounded crown. Furthermore, as described above, distal end 104 includes a trocar tip which may be composed of facets 106 separated by edges 118. The distal end 104 includes a sharpened tip 116.

The proximal end 100 is configured such that a distal biasing force applied to the anchor 99f that is positioned such that the tip 116 is adjacent an underlying bone will cause the tip 116 to cut or penetrate the underlying bone. The intermediate region 108 of the bone anchor 99f, and thus the shaft 113, includes an unthreaded portion 180 that is devoid of threads. It should be appreciated that the unthreaded portion 180 can extend along the shaft 113 from the tip 116 to any location along the shaft, up to the head 103. Thus, the shaft 113 includes a thread 126 extending distally from any location distal of the head 103 that terminates at location proximal of the tip 116, such that the unthreaded portion extends from the thread 126 to the tip 116. It should be further appreciated that the unthreaded portion 180 can include alternative fastening structure, such as ridges such as ridges 122, teeth, spikes, or the like. Thus, the user can drive the anchor 99f into the underlying bone so as to insert at least a portion of the anchor (e.g., the tip 116 and the non-threaded portion of the shaft 113 intermediate region 108) longitudinally in the bone without applying any twisting or torquing force about the longitudinal axis L to the bone anchor 99f. Once the thread 126 has reached the underlying bone, the user can then apply a torquing (i.e., screwing) force about the longitudinal axis L to the bone anchor 99f so as to seat the bone thread 128 in the bone and to then engage the locking thread 102 with a complementary thread on an implant component (not shown) into which the anchor has been inserted, as described above.

The bone thread 126 may, as shown by Fig. 6B, be tapered and rounded in cross-section. As illustrated in Fig. 6D, the bone thread 126 may also be triangular in cross section. Such a conformation may enable the anchor to more efficiently seat in bone and to resist pull-out forces once the anchor has been situated in the bone. The anchor 99f includes an unthreaded region 180. The unthreaded region 180 lacks threads, but may include ridges, teeth, spikes, and the like. As shown, the anchor 99f includes a shaft 113 that includes a threaded region 126 and an unthreaded region 180.

The helical thread region 126 or a stepped thread region or even a ridged region may occupy less than the entire length of the intermediate portion, as shown in Fig. 6B. The thread or ridges is disposed such that a region of the intermediate region adjacent to the tip of the bone anchor is smooth, and free of thread or ridges. This in turn enables the user to at least partially install the bone anchor by driving the member into the vertebral (or other) body without also having to apply a torquing force to advance the bone anchor into the body.

The thread may be of constant or varying pitch. The thread may also vary in height along its length. For example, the thread closer to the tip of the anchor may have a comparatively low height, and then transition to a taller thread closer to the proximal end of the anchor.

The distal end 104 of the anchor is suitably configured as a trocar tip. Such a tip includes facets 106 that are separated by edges 118. The end 104 suitably has a tip 116 that is sharpened so as to be capable of penetrating or cutting bone when a force is applied to bias the anchor against the bone. The tip may be slightly blunted or flattened so as to achieve a particular penetration profile.

Referring to Figs. 7-9, a bone implant assembly 215 includes a bone implant 200, such as an intervertebral implant configured to be implanted in an intervertebral disc space between a pair of adjacent vertebrae, and a plurality of bone anchors 99 of the type described above. It is to be understood that the anchor 99 shown in described implant systems is exemplary, and that the systems may include any of the disclosed anchors 99a, 99b, 99c, 99d, 99e, 99f, 99g, and any variations thereof.

The bone implant 200 includes a spacer 208 and a fixation plate 216 configured to attach to the spacer 208. The implant 200 may further include a blocking plate 232 and a locking screw 238 as illustrated. The head of the bone anchor can be configured to lock the anchors 99 into the fixation plate 216 in the manner described above.

One or more bone anchors 99 may be utilized to securely anchor an assembled configuration of the intervertebral implant 200 within an intervertebral space between adjacent vertebral bodies. Unless otherwise indicated, the intervertebral implant 200 and its components can be manufactured from any suitable biocompatible material known in the art including but not limited to titanium, titanium alloy such as TAN, stainless steel, reinforced plastics, allograft bone, and the like.

The spacer 208 defines a posterior side 208a, an anterior side 208b opposite the posterior side, lateral sides 208c, an upper surface 208d, and a lower surface 208e opposite the upper surface. In one example embodiment, a portion of the posterior side 208a between the lateral sides 208c may be curved inwardly in the direction of the anterior side 208b, defining a rounded, generally rectangular kidney-like footprint. The posterior side 208a can have a height (as measured from the tops of teeth or ridges present on the upper or lower surfaces of the spacer) in the range of from about 5 to about 20 mm, or from about 8 to about 18 mm, or even from about 10 to about 15 mm. The height (measured from the tops of teeth or ridges present on the spacer) of the anterior side 208b can be in the range of from about 8 mm to about 25 mm, or from about 10 mm to about 20 mm, or even from about 12 mm to about 15 mm. Furthermore, the height of the anterior side can be greater than that of the posterior side.

In an alternative embodiment, a portion of the posterior side 208a between the lateral sides 208c may be curved outwardly in a direction away from the anterior side 208b. In yet another alternative embodiment, the posterior side 208a may be substantially straight between the lateral sides 208c, defining a rounded, generally rectangular footprint.

The spacer 208 may have a central bore 210 formed therethrough, the shape of which substantially conforms to the footprint of the spacer 208 (e.g., a rounded, generally rectangular kidney-like footprint, or a rounded, generally rectangular footprint, depending upon the geometry of the posterior side 208a). The central bore 210 can be filled with bone growth inducing substances to allow bony ingrowth and to assist in fusion between the spacer 208 and adjacent vertebral bodies.

In an example embodiment of the spacer 208, the opposed upper and lower surfaces 208d and 208e define bone-engaging surfaces that may have gripping features 208h such as teeth, spikes, or other similar structures, formed thereon and configured to facilitate gripping engagement between the upper and lower surfaces 208d and 208e and the end plates of adjacent vertebral bodies. The teeth 214 may be pyramidal, saw toothed or other similar shapes. In alternative embodiments of the spacer 208, portions of and/or the entirety of the upper and lower surfaces 208d and 208e may be substantially smooth and devoid of any protrusions.

Upper and lower edges 208f and 208g, defined where the upper and lower surfaces 208d and 208e intersect with the posterior, anterior, and lateral sides 208a, 208b, and 208c respectively around the outer perimeter of the spacer 208, may be rounded.

In one example embodiment, the upper and lower edges 208f and 208g may be rounded using a uniform radius of curvature around the perimeter of the implant. In an alternative embodiment, the upper and lower edges 208f and 208g may be rounded using a non-uniform radius of curvature around the perimeter of the implant. In another alternative embodiment, the upper and lower edges 208f and 208g along the anterior side 208b may be rounded with a greater radius than the remainder of the upper and lower edges 208f and 208g, such that a bull nose outer surface is created on the anterior side 208b of the implant. Rounding upper and lower edges 208f and 208g may facilitate easier insertion of the spacer 208, for example by minimizing distraction of the end plates of adjacent vertebral bodies.

In an example embodiment, the spacer 208 has a generally wedge-shaped sideview profile. This wedge shape is suitably defined by a gradual decrease in the height of the spacer 208 (as measured between the upper and lower surfaces 208d and 208e) extending between the posterior side 208a in the direction of the anterior side 208b. The spacer 208 has a generally constant height between lateral sides 208c. In alternative embodiments, the spacer 208 may have a gradual increase in height followed by a gradual decrease in height extending from one lateral side 208c to the other, and/or may have a generally constant height between the posterior and anterior sides 208a and 208b, or may have convex and/or concave upper and lower surfaces 208d and 208e, thereby defining a gradual increase in height followed by a gradual decrease in height extending from the posterior side 208a to the anterior side 208b and from one lateral side 208c to the other.

A plurality of grooves or indentations 212 may be formed within the spacer 208 where the upper and lower surfaces 208d and 208e intersect with the anterior side 208b. The grooves 212 may be concave and may be configured to align with apertures 228 that extend through an anterior side 218a of the fixation plate 216 when the spacer 208 and the fixation plate 216 are in an assembled configuration. In an example embodiment, the grooves 212 may be substantially smooth and free of protrusions. Retaining grooves 214 may be formed within the lateral sides 208c of the spacer 208 between the upper and lower surfaces 208d and 208e. The retaining grooves 214 may be configured to engage complementary engaging ribs 220 of the fixation plate 216.

The fixation plate 216 is suitably defined by a generally C-shaped, channel-like body 218 that includes an anterior side 218a with upper and lower sides 218b and 218c opposite each other, and lateral sides (which may be termed "arms") 218d extending from opposite sides of the anterior side 218a in a generally perpendicular direction from the anterior side 218a. The anterior, upper, lower, and lateral sides 218a, 218b, 218c, and 218d may form a generally channel-like structure (in essence, a cradle) which may be configured to receive the anterior side 208b and at least a portion of the lateral sides 208c in partial nested engagement. As such, the upper and lower sides 208b and 208c may define gradual increases and/or decreases in height in a posterior direction from the anterior side 218a and/or between the lateral sides 208d, in order to generally conform the fixation plate 216 to the geometry of the spacer 208. The lateral sides 218d may have engaging ribs 220 formed thereon at the ends opposite the anterior side 218a, the engaging ribs 220 configured to be releasably received within the retaining grooves 214 of the spacer 208.

The anterior side 218a of the fixation plate 216 may have apertures 222 formed therethrough configured to receive grasping features of a delivery instrument. As shown, a bone anchor 99 suitably has a length greater than the length of an aperture. In an example embodiment, the apertures 222 may be substantially D-shaped. Any other aperture shape may, however, be defined as appropriate. The apertures 222 may have a retaining rib 224 formed therein configured to engage with a complimentary grasping rib of a delivery instrument. The anterior side 218a of the fixation plate 216 may also have a central bore 226 formed therethrough having an inner surface 226a with threads configured to engage complimentary threads of a locking screw 238. The anterior side 218a of the fixation plate 216 may also have a concave recess 230 formed therein configured to receive a complimentary convex surface 234d of the blocking plate 232. The recess may matably engage with the blocking plate 232.

The anterior side 218a of the fixation plate 216 may also have a plurality of apertures 228 formed therethrough configured to receive the bone anchors 99 and to define an insertion trajectory for the bone anchors. In an example embodiment, the apertures 228 may have a generally uniform cross sectional geometry configured to closely conform to the cross sectional geometry of the bone anchor 99. The apertures 228 may also include an interior thread that engages with an external disposed on the proximal end (head) of the bone anchor.

The apertures 228 may be dimensioned such that the proximal end of the bone anchor is flush with the surface 230 (or 218a) of the fixation plate when the anchor is fully installed, although this flush orientation is not necessary. The aperture 228 may also be configured such that the proximal end of the anchor is sunken below the surface of the fixation plate when the anchor is fully installed; the end of the anchor may also protrude from the fixation plate.

The apertures 228 may be disposed about the optional central bore 226 in any desired configuration and may define any insertion trajectories as appropriate. In the example embodiment depicted in Figs. 7-9, the apertures 228 are formed in opposing quadrants around the central bore 226, with two apertures 228 located near the upper side 218b and defining two generally cranial insertion trajectories, and two apertures 228 located near the lower side 218c and defining two generally caudal insertion trajectories. This configuration of aperture 228 locations and bone anchor 99 insertion trajectories is merely an example, and the scope of the instant disclosure should not be limited thereto.

An optional blocking plate 232 is shown; such plates are not a requirement, as the anchors 99 may be capable of securing the implant structure to the underlying bone without the assistance of a blocking or other structure. The plate 232 is defined by a generally disc-shaped body 234 with planar upper and lower surfaces 234a and 234b, an anterior surface 234c, and a posterior surface 234d. The upper and lower surfaces 234a and 234b and the height of the body 234 (as measured between the upper and lower surfaces 234a and 234b) may be defined to match the height (as measured between the upper and lower surfaces 218b and 218c) of the anterior side 218a of the fixation plate 216 when the blocking plate 232 is in a fully assembled configuration. The anterior surface 234c of the body 234 may be generally planar, or may be defined to match the outer surface of the anterior side 218a of the fixation plate 216 when the blocking plate 232 is in a fully assembled configuration.

The posterior surface 234d may be defined as a convex surface configured to engage with the concave recess 230 formed in the anterior side 218a of the fixation plate 216 when the blocking plate 232 is in a fully assembled configuration. The body 234 may have an aperture 236 formed therethrough. In an example embodiment, the diameter of the aperture may be slightly larger than the diameter of the central bore 226 of the fixation plate 216, such that a locking screw 238 may be inserted into the aperture with no interference therebetween. In another embodiment, the diameter of the aperture 236 may be substantially the same as that of the central bore 226, and the inner surface of the aperture may have threads formed thereon, the threads configured to engage complimentary threads of the locking screw 238. The aperture 236 may further be defined by a concave recess 236a formed within the anterior surface 234c, the concave recess 236a configured to receive the convex head 242 of the locking screw 238.

The optional locking screw 238 includes a shaft 240 that defines longitudinally opposing proximal and distal ends 240a and 240b, respectively, and a head 242 coupled to the proximal end 240a of the shaft 240, either directly or indirectly via an unthreaded neck 244 that is coupled between the proximal end 240a of the shaft 240 and the head 242. The head 242 can define a generally convex shape between the interface of the head 242 and the neck 244 that extends outward towards a proximal end 242a of the head 242. The convex shape of the head may be configured to engage the concave recess 236a of the blocking plate 232. The head 242 can assume any other suitable alternative shape as appropriate. Helical threads 246 extend radially out from the shaft 240 at locations at and between the proximal and distal ends 240a and 240b that are configured to engage complementary threads on the inner surface 226a of the central bore 226 of the fixation plate 216. Thus, a substantial entirety of the shaft 240 between the proximal and distal ends 240a and 240b may be threaded. The distal end 242a of the head 242 may have driving features 242b defined therein, designed to engage with complementary driving features of a delivery instrument.

During operation, the spacer 208 is seated within the fixation plate 216 such that the retaining ribs engage with the retaining grooves on the lateral sides of the spacer 208. Four bone anchors 99 are inserted through corresponding grooves within the fixation plate 216, and have been driven to an essentially fully inserted position. In this embodiment, the heads of the bone anchors 99 may be flush with the surface of the fixation plate 216. The fixation plate may include an aperture that is configured to releasably engage with a delivery instrument, which instrument may include an armature or other extension that engages with the fixation plate. The heads of the anchors may, as described elsewhere herein, include a thread that engages with a complementary thread of

Fig. 9 depicts an example embodiment of the intervertebral implant 200 partially assembled inside of an intervertebral space between adjacent vertebral bodies V6 and V7 (the blocking plate and locking screw have been omitted for simplicity). As an initial step, the spacer 208 has been prepared for insertion, for example by being packed with bone growth inducing substance and or/having its outer surfaces properly prepared. The spacer 208 has also been seated within the fixation plate 216 such the retaining ribs are seated with the retaining grooves on the lateral sides of the spacer 208. The spacer 208 is then inserted into the intervertebral space between the adjacent vertebral bodies V6 and V7 using a delivery instrument. An instrument is then used to deliver the four bone anchors 99 into the grooves in the fixation plate and drive them into an almost fully inserted position.

If a blocking plate and locking screw are used, an instrument is used to drive the bone anchors 99 into their fully inserted position in the manner described above, and the blocking plate is received within the concave recess in the anterior side of the fixation plate, and the locking screw would be driven into the central bore of the fixation plate and finally tightened, thereby blocking the bone anchors 99 from backing out of the assembled intervertebral implant 200.

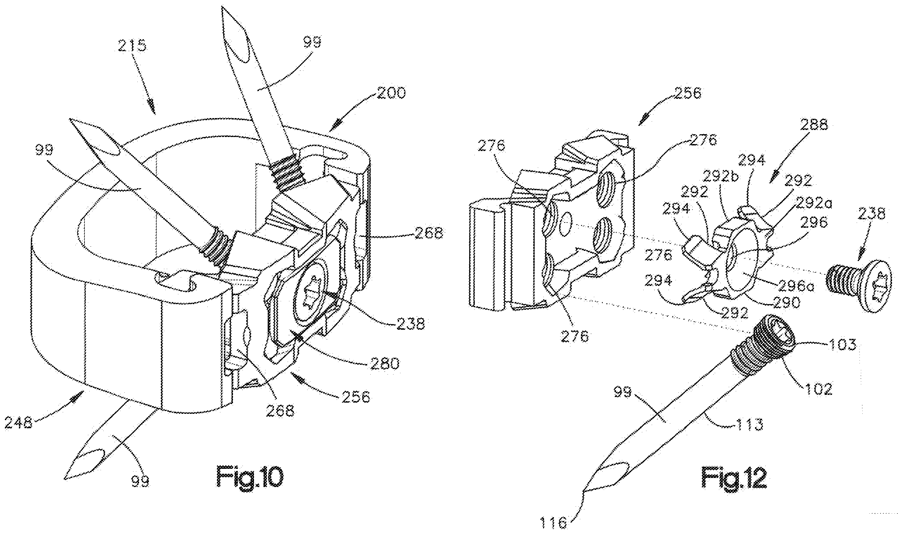

It should be appreciated that the intervertebral implant 200 can be alternatively constructed as desired. For instance, referring now to Fig. 10-13D, the fixation plate 256 is defined by a generally rectangular body 258 that includes an anterior side 258a and lateral sides 258b extending therefrom, the lateral sides 258b configured to engage with the retaining grooves 252 of the spacer 248. In an example embodiment, the lateral sides 258b are generally J-shaped, extending initially from opposite sides of the anterior side 258a in a direction perpendicular to and away from the anterior side 258a, and through curved sections 258c before returning in a direction perpendicular to and towards the anterior side 258a and terminating in distal ends 258d. It should be noted that this configuration for lateral sides 258b is merely an example, and any other geometry may be used as appropriate.

Upper and lower edges of the anterior side 258a, defined where upper and lower surfaces 258e and 258f of the anterior side intersect with an anterior surface 258g of the anterior side, may be rounded. In an example embodiment, the upper and lower edges 258e and 258f may be rounded using a uniform radius of curvature. In an alternative embodiment, the upper and lower edges 258e and 258f may be rounded using a non-uniform radius of curvature. Rounding upper and lower edges 258e and 258f may facilitate easier insertion of the fixation plate 256, for example by minimizing distraction of the end plates of adjacent vertebral bodies.

The lateral sides 258b may have retaining ribs 260 formed thereon at the distal ends 258d, the retaining ribs 260 configured to be releasably received within the retaining grooves 252 of the intervertebral implant 258. Access grooves 262 and 264 may be formed within the retaining ribs 260 and the lateral sides 258b, in the area where the lateral sides 258b interface with the anterior side 258a, respectively. The access grooves 262 and 264 may be configured to align with complimentary access grooves 254 of the spacer 248, thereby defining an access cavity 268 for receiving an engaging feature of a delivery instrument when the spacer248 and the fixation plate 256 are in an assembled configuration. The access grooves 264 may have a retaining shelf 266 formed therein configured to engage with an engaging feature of a delivery instrument, for example the raised ribs 258d formed on the insertion rods 258 of the delivery instrument 278, described in greater detail below. The lateral sides 258b may also have bores 278 formed within the curved sections 258c, the apertures configured to receive, for example the distal engagement tips 258c of the insertion rods of 258 of the delivery instrument 278.

The anterior side 258a of the fixation plate 256 may have gripping grooves 268 formed within the upper and lower surfaces 258e and 258f of the anterior side 258a, the gripping grooves 268 configured to receive grasping arms of a delivery instrument. The gripping grooves 268 may have a gripping ridge 270 formed therein, the gripping ridge configured to be engaged by the complimentary grasping features formed at the ends of the grasping arms of the delivery instrument. The anterior side 258a of the fixation plate 256 may also have a recess 272 formed therein configured to receive additional components of the intervertebral implant 200, for example a ratchet blade 288, a blocking plate 280, or the like. The anterior side 258a may also have a central bore 274 formed therethrough having an inner surface 274a with threads configured to engage complimentary threads of a locking screw 238. In an example embodiment, the central bore 274 may be formed within the recess 272.

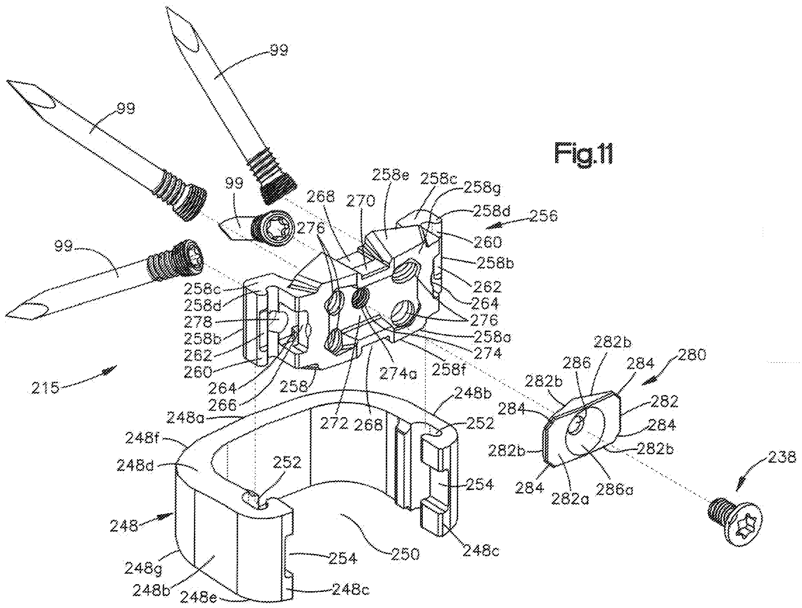

The anterior side 258a of the fixation plate 256 may also have a plurality of apertures 276 formed therethrough configured to slidably receive the bone anchors 99 and to define an insertion trajectory for each of the bone anchors 99; as shown in Fig. 11, the grooves may include interior threads adapted to engage complementary threads on the anchors. Alternatively, a bone anchor may also include splines or flanges that engage with the fixation plate so as to maintain the bone anchor in position. The bone anchor is suitably installed by way of a driving instrument that applies a force to bias the tip of the at least one bone anchor into a vertebral body.

In an example embodiment, the apertures 276 may have a generally uniform cross sectional geometry configured to closely conform to the cross sectional geometry of the body of the bone anchor 99 between the head and the distal end.

When a bone anchor 99 is in a fully inserted position within a respective aperture 276, the surface of the head of the fixation device may be flush with the outer surface of the anterior side 258a of the fixation plate 256. The head may also protrude, in some embodiments, from the surface of the fixation plate.

The apertures 276 may be disposed about the central bore 274 in any desired configuration and may define any insertion trajectories as appropriate.

The blocking plate 280 is defined by a generally rectangular body 282 with an anterior surface 282a, and a plurality of angled posterior surfaces 282b generally opposite the anterior surface 282a. The body 282 may have an aperture 286 formed therethrough In an example embodiment, the diameter of the aperture may be slightly larger than the diameter of the central bore 274 of the fixation plate 256, such that a locking screw 238 may be inserted into the aperture with no interference therebetween.

In another embodiment, the diameter of the aperture 286 may be substantially the same as that of the central bore 274, and the inner surface of the aperture may have threads formed thereon, the threads configured to engage complimentary threads of the locking screw 238. The aperture 286 may further be defined by a concave recess 286a formed within the anterior surface 282a, the concave recess 286a configured to receive the convex head 242 of the locking screw 238.

The height, width, and depth of the body 282 may be proportioned so that the blocking plate 280 will be received within the recess 272 of the fixation plate 256, such that the anterior surface 282a of the body 282 is substantially flush with the anterior surface 258g of the anterior side 258a of the fixation plate 256 when the fixation plate 256 and the blocking plate 280 are in an assembled configuration. The anterior surface 282a of the body 282 may be generally planar, or may be defined to match the outer surface of the anterior side 258a of the fixation plate 256 when the blocking plate 280 and the fixation plate 256 are in a fully assembled configuration.

In an example embodiment wherein the blocking plate 280 and locking screw 238 are installed after the spacer 248 and fixation plate 256 have been inserted into an intervertebral space and the bone anchors 99 driven into their fully inserted positions, the angled posterior surfaces 282b and chamfered corners 284 of the blocking plate 280 may be configured to engage the heads of the bone anchors 99 within the recess 272 of the fixation plate 256 when the blocking plate 280 is installed followed by the locking screw 238. When final tightening of the locking screw 238 is performed, the blocking plate 280 may rigidly fix the bone anchors in position, and additionally prevent pullout of the members.

In another exemplary, non-limiting embodiment wherein the spacer 248, the fixation plate 256, the blocking plate 280, and the locking screw 238 are pre-assembled, but not finally tightened, and then inserted into an intervertebral space before the bone anchors 12C are inserted and driven into position, the angled posterior surfaces 282b and chamfered corners 284 of the blocking plate 280 may be configured to allow the bone anchors to be inserted and driven into position with the blocking plate 280 and the locking screw 238 in place.

In this embodiment, the angled posterior surfaces 282b may have wedge features formed thereon that are configured to interfere between the heads of the bone anchors and the surrounding structure of the fixation plate 256, for example by applying outward force laterally upward and downward on the bone anchors 99 to lock them in place when final tightening is applied to the locking screw, and additionally to prevent pullout of the bone anchors.

Referring now to Fig. 10, an example embodiment of the intervertebral implant 200 in a completely assembled configuration outside of an intervertebral space is shown. The fixation plate 256 has been engaged with the spacer 248 such that the retaining ribs of the fixation plate 256 are seated with the retaining grooves of the spacer 248. Four bone anchors 99 have been inserted through corresponding grooves within the fixation plate 256, and have been driven to a fully inserted position. The blocking plate 280 and the locking screw 238 have been installed and finally tightened.

Referring to Fig. 12, a fixation plate 256 includes a plurality of apertures 276 that extend therethrough, the grooves being configured to accept bone anchors 99 inserted therethrough. If desired, the system also can also include a ratchet plate 288, which plate is assembled into the fixation plate 256 using a locking screw 238. The bone anchor 99 suitably includes a locking thread disposed on the outside of the head of the anchor, which thread is adapted to engage with a thread disposed on the interior of aperture 276 so as to lock the anchor into place.

Another embodiment is shown in Figures 15a-15e. As shown in Figure 15a, an implant assembly 200 includes a spacer 208 and a fixation plate 216. The fixation plate is suitably made from titanium, although other metals, polymers, or composite materials are suitable. The spacer is suitably made from PEEK or other polymers.

The fixation plate 216 suitably includes a bore hole 216, which hole may be adapted to receive or otherwise engage an installation instrument, an aiming device, or both. The apertures 228 in the fixation plate 216 suitably include internal threads (shown), which threads are adapted to engage complementary threads on an anchor 99.

The spacer 208 suitably defines a posterior side 208a, an anterior side 208b opposite the posterior side, lateral sides 208c, an upper surface 208d, and a lower surface 208e opposite the upper surface. In one example embodiment, a portion of the posterior side 208a between the lateral sides 208c may be curved inwardly in the direction of the anterior side 208b, defining a rounded, generally rectangular kidney-like footprint. The implant may include a somewhat curved or J-shaped arm 252, which arm is configured to engage with a complementary feature 220 on the fixation plate.

The spacer 208 may optionally include a side channel 210a, which side channel may be packed with a bone growth inducing substance or other material. The spacer may include one, two, or more side channels. The implant 200 may optionally include a side aperture 210b, which channel allows material to pass into or out of channel 210a. The implant 200 also suitably includes a central channel 210, which central channel may be packed with bone growth material, if desired. A central aperture 226b may be present, which central aperture allows material to pass into or out of the central channel 210. The central channel and aperture are optional. The spacer 208 may include cutouts 212 that align with the apertures 228 of the fixation plate 216 when the fixation plate is installed with the spacer 208.

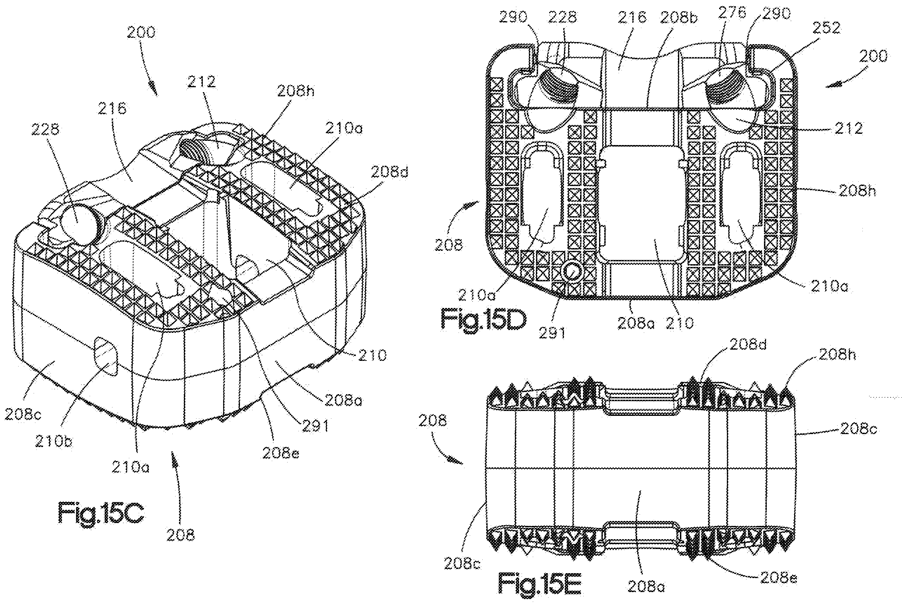

The implant 200 may also include strips 290. These strips suitably extend from the surface of the implant 200, and act to promote effective engagement between the implant 200 and the fixation plate 216. As shown in Figure 15a, the strips 290 may have sloped or wedged edges so as to facilitate slidably engaging the implant 200 and the fixation plate 216. Once the fixation plate and implant are engaged, the strips 290 act as shims or braces so as to more tightly engage the fixation plate with the implant; as shown in Figure 15d, the strips may wedge between the fixation plate 216 and the spacer 208.

Figure 15b is a side-on view of spacer 208. This view shows side aperture 210a, teeth 208h, and lateral side 208c.

Figure 15c illustrates an alternative view of the implant 200, in which figure the fixation plate and spacer are in an assembled configuration. As shown in this figure, the cutouts 212 align with the apertures 228 of the fixation plate. The arms 252 of the spacer 208 are configured to slidably engage with the complementary features of the fixation plate so as to guide and maintain the fixation plate 216 in position..

Figure 15d is a top view of the implant 200 in its assembled configuration. The side channels 210a and central channel 210 are shown; as described elsewhere herein, the channels can be packed with bone growth materials. Marker 291 is suitably an x-ray-visible material disposed within the spacer 208. This may be accomplished by forming a hole or recess in the spacer and then inserting the marker material (e.g., titanium) into the hole or recess. Figure 15d also illustrates the strips 290 acting as shims to maintain fixation plate 216 in position.

Figure 15e is a posterior view of the implant 200, showing the posterior side 208a, the lateral side 208c, the upper surface 208d, and teeth 208h.

In some embodiments, the implant 200 is a single body. Such unitary bodies may be made of titanium, PEEK or other materials. Figure 15c illustrates the configuration of such a single body; such a single body would integrate fixation plate 216 and spacer 208 into a single body; i.e., the fixation plate and spacer 208 are integrated into a single body and are not separate from one another.

It should be noted that although the description and accompanying Figures. illustrating the intervertebral implant included herein depict example embodiments of the intervertebral implant that include four bone anchors, with two of the four bone anchors having a generally cranial insertion trajectory and the remaining two bone anchors having a generally caudal insertion trajectory (e.g., Fig. 7). Other configurations of the intervertebral implant using more or fewer bone anchors and/or varying insertion trajectories are possible and intended to be included within the scope of the instant disclosure. For example, in an alternative embodiment of the intervertebral implant, the fixation plate may have three grooves formed therein having any desirable placement and/or insertion trajectory with respect to the central bore (e.g., with two of the three bone anchors having a generally caudal insertion trajectory and the remaining bone anchor having a generally cranial insertion trajectory, or with two of the three bone anchors having a generally cranial insertion trajectory and the remaining bone anchor having a generally caudal insertion trajectory).

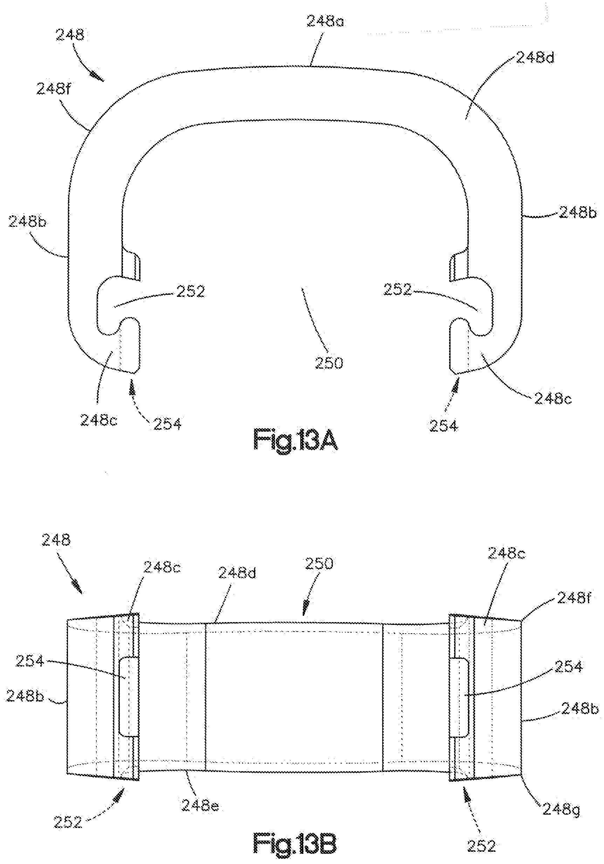

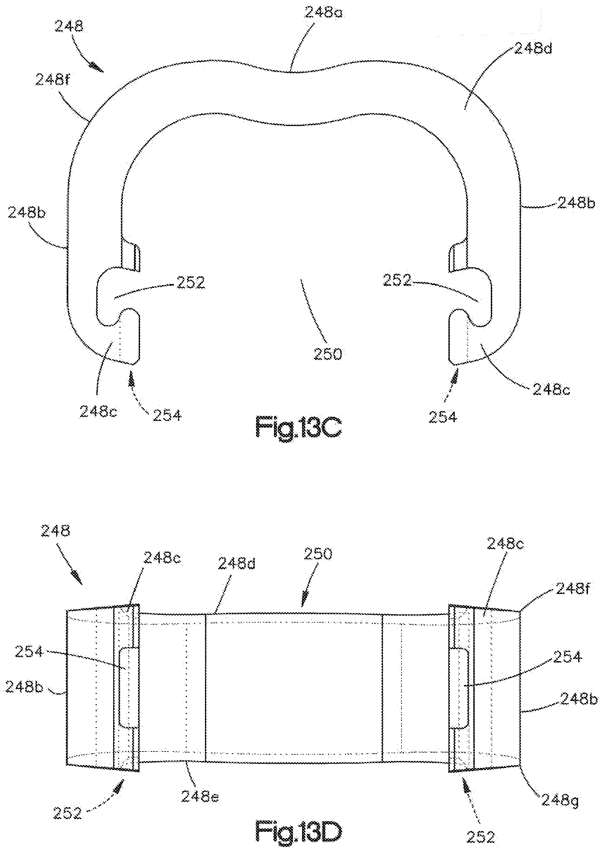

Referring to Figs. 13A-D the spacer 248 has a generally C-shaped footprint defined by a posterior side 248a, lateral sides 248b terminating in distal ends 248c opposite the posterior side 248a, an upper surface 248d, and a lower surface 248e opposite the upper surface. In an example embodiment, a portion of the posterior side 248a between the lateral sides 248b may be curved inwardly in a direction toward the distal ends 248c, as depicted in Figs. 13C and 13D. In an alternative embodiment, a portion of the posterior side 248a between the lateral sides 248b may be curved outwardly in a direction away from the distal ends 248c. In another alternative embodiment, the posterior side 248a may be substantially straight between the lateral sides 248b, as depicted in Figs. 13A and 13B. The posterior side 248a and lateral sides 248b define an open central bore 250, the shape of which substantially conforms to the footprint of the spacer 248. The central bore 250 can be filled with bone growth inducing substances to allow bony ingrowth and to assist in fusion between the spacer 248 and adjacent vertebral bodies.

In an example embodiment of the spacer 248, the upper and lower surfaces 248d and 248e may have gripping features such as teeth, spikes, or similar structures formed thereon and configured to facilitate gripping engagement between the upper and lower surfaces 248d and 248e and the end plates of adjacent vertebral bodies. The teeth may be pyramidal, saw toothed or other similar shapes. In alternative embodiments of the spacer 248, portions of and/or the entirety of the upper and lower surfaces 248d and 248e may be substantially smooth and devoid of any protrusions. Upper and lower edges 248f and 248g, defined where the upper and lower surfaces 248d and 248e intersect with the posterior and lateral sides 248a and 248b respectively around the perimeter of the spacer 248, may be rounded. In an example embodiment, the upper and lower edges 248f and 248g may be rounded using a uniform radius of curvature around the perimeter of the implant. In an alternative embodiment, the upper and lower edges 248f and 248g may be rounded using a non-uniform radius of curvature around the perimeter of the implant. Rounding upper and lower edges 248f and 248g may facilitate easier insertion of the spacer 248, for example by minimizing distraction of the end plates of adjacent vertebral bodies.

In an example embodiment, the spacer 248 has a generally wedge-shaped sideview profile. This wedge shape is defined by a gradual increase in the height of the spacer 248 (as measured between the upper and lower surfaces 248d and 248e) extending outwardly in a direction away the posterior side 248a in the direction of the distal ends 248c. The spacer 248 has a generally constant height between lateral sides 248b. In alternative embodiments, the spacer 248 may have a gradual increase in height followed by a gradual decrease in height extending from one lateral side 248b to the other, and/or may have a generally constant height between the posterior sides 248a and the distal ends 248c, or may have convex and/or concave upper and lower surfaces 248d and 248e, thereby defining a gradual increase in height followed by a gradual decrease in height extending from the posterior side 248a to the distal ends 248c and from one lateral side 248b to the other.

Retaining grooves 252 may be formed within the distal ends 248c of the spacer 248, for example in a vertical direction substantially perpendicular to a horizontal midplane defined between the upper and lower surfaces 248d and 248e. The retaining grooves 252 may be configured to releasably engage complementary retaining ribs 260 of the fixation plate 256. The distal ends 248c may also have access grooves 254 formed therein between the upper and lower surfaces 248d and 248e. The access grooves 254 may be configured to align with complimentary access grooves of the fixation plate 256.

The intervertebral implant may of course have matching grooves formed therein. Such alternative embodiments with two bone anchors having one of a generally cranial or caudal trajectory and a third bone anchor having the opposite general trajectory may allow for the stacking of two or more assembled configurations of the intervertebral implant in place of adjacent vertebral bodies removed from an intervertebral space. Additionally, while the bone anchors illustrated in the various Figures herein generally have divergent insertion trajectories with respect to each other, fixation plates may also be configured so that one or more of the bone anchors will have convergent insertion trajectories with respect to each other, or similar insertion trajectories (e.g., laterally towards a common side).

For example, bone anchors with generally cranial insertion trajectories may converge toward one another, may diverge away from one another, or may both follow similar insertion trajectories, while bone anchors with generally caudal insertion trajectories may converge toward one another, may diverge away from one another, or may both follow similar insertion trajectories. Any combination of the above insertion trajectory configurations may be used as appropriate.

One exemplary method of installing the claimed anchors is now described, with reference to Fig. 2 and Figs. 10-13D; these figures are illustrative only and the disclosed installation technique is suitable for other disclosed anchors. The spacer 248 is suitably assembled with the fixation plate 256 and is installed into the appropriate location in the patient's spine. One suitable technique for performing this installation is described in literature associated with the Synthes Synfix™ system (www.synthes.com), which technique is incorporated herein by reference for all purposes. Once the assembly of the implant and fixation plate are installed in the patient, the user inserts a bone anchor 99 into groove or aperture 276, which is suitably configured to define an insertion trajectory for the bone anchor 99. The aperture 276 is suitably frustoconical in configuration and includes an interior thread complementary to the thread 102 disposed on the head of the bone anchor 99.

Insertion of the anchor into the aperture 276 may be accomplished manually or with mechanical assistance. In some embodiments, an aiming component is used to assist with alignment and insertion of the anchor. The aiming component may include a bore or groove to maintain the anchor in proper orientation or alignment relative to the aperture 276 into which the anchor is inserted.

The aiming component may be configured so as to engage with the fixation plate so as to provide a stable platform for anchor insertion. For example, the aiming component may include a protrusion or tab that engages with a complementary recess, aperture, or slot on the fixation plate so as to maintain the aiming component's position. The aiming component may also relasably or rotatably mount to the fixation plate. The aiming component may be configured to support insertion of a single anchor at a time, or even two or more anchors.

In one exemplary embodiment, the user inserts the anchor into the aperture 276 until the tip of the anchor contacts one of the vertebrae that is the subject of the implantation process. Once the anchor tip contacts the bone, the user may effect an awling motion on the anchor so as to bias the tip into the vertebral bone and to drive the anchor more deeply into the bone. The awling motion may be a twisting back-and-forth motion (e.g., torquing) accompanied by applying a force in the direction of the tip of the anchor so as to bias the tip into the bone during awling.

In another embodiment, the user forms, with an awl or other implement, a hole (which may be termed a pilot hole) in the vertebral body. The user then inserts the anchor into this hole. As described elsewhere herein, the anchor may (e.g., Fig. 5) include a thread of variable height (or even variable pitch), that runs from a lower height at the distal end to a higher (taller) height closer to the proximal end of the anchor.

The awl is usually of the same or a similar configuration as the minor diameter of the tapered anchor. In this way, when the user inserts the anchor into the pilot hole, at least a portion of the anchor is present within the hole when the tip of the anchor makes contact with the end of the hole. By then applying a twisting force to the anchor, the user can seat all of the threads of the anchor within the bone by using fewer turns than would be needed to seat every thread of the anchor if the anchor had to penetrate the bone starting with its tip.

For instance, as illustrated in Figs. 14A-B, the user may form a pilot hole into the underlying bone for use with assembly system 215. By reference to Figs. 14A and 14B, the user may in some embodiments form a pilot hole 117 into underlying bone 119, such that the pilot hole 117 conforms to or is similar to the minor diameter of the bone anchor 99g shown in Fig. 14A. The pilot hole may be formed with an awl, drill, or other similar implement. The pilot hole is not necessary in all embodiments, as the anchor 99 may be configured to pierce and engage with bone without the assistance of a pilot hole.

The user may then insert an anchor 99g through the fixation plate (not labeled) into the pilot hole 117. The pilot hole 117 is suitably sized such that the threads of the anchor 99g contact the interior of the underlying bone 119 that defines the pilot hole 117 while the anchor 99g is partially inserted in the hole 117, thereby preventing the anchor 99g from being further inserted into the pilot hole 117 without application of a force to seat the threads 122 in the underlying bone 119.

The user may then applying a screwing or torquing motion to the bone anchor 99g, so as to engage additional threads 122 in the bone 119, as shown in Fig. 14B. Further torquing may then be applied to seat additional threads in the bone material, as shown in Fig. 14C. Fig. 14D illustrates the fully-seated anchor 99g. As shown by the progression of Figs. 14B-14D, the anchor 99g enables a user to seat the anchor and engage all of the anchor's threads with fewer turns than would be needed to seat every thread of the anchor if the anchor had to penetrate the bone starting with its tip. This in turn can speed the installation of the anchor.

In other embodiments, the assembly 115 includes the bone anchor 99g seated in the pilot hole 117 may include ridges along the length of the anchor, such as the shaft 103. In some embodiments, the ridges are of varying height, going from a lower height at the tip to a taller height at the proximal end. The ridges may also be of the same height along the length of the anchor. With this configuration, the user can seat all ridges of an anchor in bone without having to drive the entire length (i.e., seat every ridge) of the anchor into the bone. This, too, can speed installation of the anchor.

The user may, in some embodiments, drive the anchor into the subject bone by application of an impact force, such as tapping or even hammering. The modulation of such force will depend on the user's needs; such force is suitably modulated in accordance so as to avoid damaging any anatomical structures that are in the vicinity of the anchor insertion.

The user suitably applies sufficient force for a sufficient time that the proximal end and external thread 102 of the anchor 99g contacts the complementarily-shaped entry of the groove. Once the threads contact one another, the user may apply a torquing force to the anchor so as to engage the external thread of the anchor with the internal thread of the aperture 276. The user then tightens the thread of the anchor so as to secure the anchor into place.

In an alternative embodiment, the anchor includes splines at the proximal end of the anchor 99. The splines may then engage with complementary splines disposed within the aperture 276. The spline-spline engagement between the anchor and aperture acts to prevent the installed anchor from rotating. The installed anchors may then be secured into place using a locking bolt and blocking plate, as described elsewhere herein. While anchor 99g is used in the cited figures for illustrative purposes, it is to be appreciated that other anchors (e.g., anchors 99a-99f) may be used in the disclosed systems.

In one procedure for installing the disclosed devices, the user performs a discectomy, which procedure may include the removal of cartilaginous endplates. A suitably sized implant assembly (e.g., elements 216 and 200 in figure 15a) is selected and inserted into the patient; this insertion may be accomplished with the use of the Synthes Quick Inserter/Distractor (SQUID™) device; www.synthes.com.

An aiming device (e.g., an aiming device associated with the Synthes SynFix™ system; www.synthes.com) may be inserted into a guide hole (e.g., element 226 on figure 15a) to assist the user with aligning the anchors 99 for installation into the implant assembly. The user may then use a driving instrument (e.g., a screwdriver, or U-joint driver) to install the first anchor 99. The awl tip 104 of the anchors 99 enables the user to insert a first anchor into the spinal bone by effecting an awling, twisting, or other back-and-forth motion while biasing the anchor into the bone. Once the anchor is inserted and the threads 102 on the head of the anchor contact the complementary threads of the apertures (e.g., element 228 in Figure 15a) of the fixation plate (e.g., element 216 in Figure 15a), the user may then use a screwdriver or other instrument to engage and lock the threaded anchors to the fixation plate. Other anchors are installed in a similar fashion.

In some embodiments, the anterior edge of the installed implant is flush to about 3 mm-recessed relative to the anterior aspect of the adjacent vertebrae. The user may assess the positioning of the implant assembly by using an x-ray or other imaging device to determine the location of the implant relative to the vertebral bodies. The fixation plate (suitably fashioned from titanium) may be visible on an x-ray. The implant may also include an x-ray marker (e.g., element 291 on Figure 15d), which is suitably titanium or other x-ray opaque material, which marker enables the user to assess the position and orientation of the implant. (The x-ray marker may be about 2 mm from the posterior edge of the implant.) The fixation plate and x-ray marker thus allow for intraoperative radiographic assessment of the installed implant.

It should be appreciated that a variety of kits can be provided that include one or more components of the fixation system or the intervertebral implant. A kit may contain multiple, identical components (e.g., multiple anchors that are the same size and configuration) or may contain different components (e.g., multiple anchors of different sizes.) In this way, a single kit may include a range of components, resulting in the kit being suitable for use with a range of patients having need for differently-sized or configured implants.

For example, within a single kit, bone anchors may be provided that have varying lengths, differing head, shaft, or tip configurations, differing cross sectional geometries, and so on, depending, for example, on the type of procedure being performed by a surgeon, or on the particular anatomies of individual patients.

The kits may also be configured differently with respect to which components of the individual systems are included in the kits. For example, a kit may include bone anchors with varying configurations and/or features, and may or may not include a device to hold the assembled implant for insertion into the subject. The kit may also include fixation rods and the like.

In another example, a kit for the intervertebral implant may include bone anchors of varying lengths and features, and may also include one or more intervertebral implants, one or more fixation plates, one or more blocking plates, one or more ratchet screws, or one or more locking screws. Example kits may also include an instrument for delivering the system into a subject, and may also include a mallet, a pinion drive, a sonic hammer, a screwdriver, or the like for biasing an anchor into a vertebral body and/or for locking the anchor into place.

Although bone anchors and the other components of the fixation and implants have been described herein with reference to preferred embodiments or preferred methods, it should be understood that the words which have been used herein are words of description and illustration, rather than words of limitation. For example, it should be appreciated that the structures and/or features of components of the fixation system may be combined with or otherwise integrated with the structures and/or features of the intervertebral implant. Although the fixation system and the intervertebral implant have been described herein with reference to particular structures, methods, and/or embodiments, the scope of the instant disclosure is not intended to be limited to those particulars, but rather is meant to extend to all structures, methods, and/or uses of the fixation system and the intervertebral implant. Those skilled in the relevant art, having the benefit of the teachings of this specification, may effect numerous modifications to the fixation system and/or the intervertebral implant as described herein, and changes may be made without departing from the scope of the invention as defined by the appended claims.

## Claims

1. A bone implant assembly (215) comprising:
an implant (200) including:
a spacer (208) configured to be inserted into an intervertebral space, and
a fixation plate (216) configured to attach to the spacer (208), the fixation plate defining at least one aperture (228) extending therethrough,
a bone anchor (99) configured for insertion into the aperture (228) so as to extend through the at least one aperture and into a bone (V6, V7), thereby affixing the implant (200) to the bone, the bone anchor including:
a proximal end (100), a distal end (104) opposite the proximal end, and an intermediate portion (108) extending therebetween, the distal end (104) defining a tip (116) having a sharpened tip point that is configured as a trocar tip, the sharpened tip point being configured to cut into the bone (V6, V7), the proximal end (100) defining a head (103), the bone anchor further including a shaft (113) that is elongate along a central longitudinal axis (L), the shaft (113) extending proximally from the tip (116) to the head (103), and at least a portion (180) of the shaft (113) being unthreaded from the tip (116) and along a portion of the intermediate portion (108) toward the proximal end (100),
wherein the shaft (113) defines an external thread (126) that extends between the head (103) and the unthreaded portion (180), the bone anchor (99) configured to extend through the at least one aperture (228) and into the bone (V6, V7) so as to fix the implant (200) to the bone, such that the external thread (126) contacts the bone (V6, V7).

2. The assembly of claim 1, in which the proximal end (100) defines a conical head.

3. The assembly of claim 1, in which at least the portion of the intermediate portion (108) further comprises a ridge, a spike, a tooth (122), or any combination thereof.

4. The assembly of claim 3, in which the intermediate portion (108) comprises a plurality of concentric ridges (122).

5. The assembly of claim 1, in which the proximal end (100) of the bone anchor (99) comprises a recess (112) or protrusion (111) adapted to engage a complementary feature of a driving instrument.

6. The assembly of claim 1, in which the unthreaded portion (180) is substantially smooth.

7. The assembly of claim 1, in which the tip (106) defines three or more facets (114).

8. The assembly of claim 1, further comprising a driving instrument configured so as apply force that biases the tip (106) of the bone anchor (99) into a vertebral body (V6, V7).

## Patentansprüche

1. Knochenimplantatsystem (215), umfassend:
ein Implantat (200), umfassend:
einen Abstandshalter (208), der ausgestaltet ist, in einen intervertebralen Raum eingesetzt zu werden, und
eine Befestigungsplatte (216), die ausgestaltet ist, an den Abstandshalter (208) befestigt zu werden, wobei die Befestigungsplatte mindestens eine Öffnung (228) bestimmt, die sich dadurch erstreckt,
einen Knochenanker (99), der ausgestaltet ist, in die Öffnung (228) eingesetzt zu werden, um sich durch die mindestens eine Öffnung und in einen Knochen (V6, V7) zu erstrecken, und dadurch das Implantat (200) am Knochen zu befestigen, wobei der Knochenanker Folgendes umfasst:
ein proximales Ende (100), ein dem proximalen Ende gegenüberliegendes distales Ende (104) und ein sich dazwischen erstreckender Zwischenabschnitt (108), wobei das distale Ende (104) eine Spitze (116) bestimmt, die einen scharf auslaufenden Spitzenpunkt aufweist, der als eine Trokarspitze ausgestaltet ist, wobei der scharf auslaufende Spitzenpunkt ausgestaltet ist, in den Knochen (V6, V7) zu schneiden, wobei das proximale Ende (100) einen Kopf (103) bestimmt, wobei der Knochenanker überdies einen Schaft (113) umfasst, der entlang einer zentralen Längsachse (L) länglich ist, wobei sich der Schaft (113) proximal von der Spitze (116) zum Kopf (103) erstreckt, und wobei mindestens ein Abschnitt (180) des Schafts (113) von der Spitze (116) und entlang eines Abschnitts des Zwischenabschnitts (108) zum proximalen Ende (100) kein Gewinde aufweist,
wobei der Schaft (113) ein externes Gewinde (126) bestimmt, das sich zwischen dem Kopf (103) und dem Abschnitt ohne Gewinde (180) erstreckt, wobei der Knochenanker (99) ausgestaltet ist, sich durch die mindestens eine Öffnung (228) und in den Knochen (V6, V7) zu erstrecken, um das Implantat (200) derart an dem Knochen zu befestigen, dass das externe Gewinde (126) den Knochen berührt (V6, V7).

2. System nach Anspruch 1, wobei das proximale Ende (100) einen konischen Kopf bestimmt.

3. System nach Anspruch 1, wobei mindestens der Abschnitt des Zwischenabschnitts (108) überdies eine Gewinderippe, eine Zinke und einen Zahn (122) oder eine Kombination daraus umfasst.

4. System nach Anspruch 3, wobei der Zwischenabschnitt (108) eine Vielzahl von konzentrischen Gewinderippen (122) umfasst.

5. System nach Anspruch 1, wobei das proximale Ende (100) des Knochenankers (99) eine Vertiefung (112) oder einen Vorsprung (111) umfasst, der/die angepasst ist, um in ein komplementäres Merkmal eines Treibinstruments einzugreifen.

6. System nach Anspruch 1, wobei der Abschnitt ohne Gewinde (180) im Wesentlichen glatt ist.

7. System nach Anspruch 1, wobei die Spitze (106) drei oder mehr Flächen (114) umfasst.

8. System nach Anspruch 1, das überdies ein Treibinstrument umfasst, das derart ausgestaltet ist, eine Kraft anzuwenden, die die Spitze (106) des Knochenankers (99) in einen Wirbelkörper (V6, V7) vorspannt.

## Revendications

1. Ensemble d'implant osseux (215) comprenant :
un implant (200) comprenant :
un dispositif d'espacement (208) configuré pour être inséré dans un espace intervertébral, et
une plaque de fixation (216) configurée pour se fixer sur le dispositif d'espacement (208), la plaque de fixation définissant au moins une ouverture (228) s'étendant à travers cette dernière,
un ancrage osseux (99) configuré pour l'insertion dans l'ouverture (228) afin de s'étendre à travers la au moins une ouverture et dans un os (V6, V7), fixant ainsi l'implant (200) sur l'os, l'ancrage osseux comprenant :
une extrémité proximale (100), une extrémité distale (104) opposée à l'extrémité proximale, et une partie intermédiaire (108) s'étendant entre l'extrémité proximale et l'extrémité distale, l'extrémité distale (104) définissant une pointe (116) ayant un point de pointe pointu qui est configuré comme une pointe de trocart, le point de pointe pointu étant configuré pour couper dans l'os (V6, V7), l'extrémité proximale (100) définissant une tête (103), l'ancrage osseux comprenant en outre un arbre (113) qui est allongé le long d'un axe longitudinal central (L), l'arbre (113) s'étendant de manière proximale de la pointe (116) à la tête (103), et au moins une partie (180) de l'arbre (113) n'étant pas filetée à partir de la pointe (116) et le long d'une partie de la partie intermédiaire (108) vers l'extrémité proximale (100),
dans lequel l'arbre (113) définit un filetage externe (126) qui s'étend entre la tête (103) et la partie non filetée (180), l'ancrage osseux (99) étant configuré pour s'étendre à travers la au moins une ouverture (228) et dans l'os (V6, V7) afin de fixer l'implant (200) sur l'os, de sorte que le filetage externe (126) est en contact avec l'os (V6, V7).

2. Ensemble selon la revendication 1, dans lequel l'extrémité proximale (100) définit une tête conique.

3. Ensemble selon la revendication 1, dans lequel au moins la partie de la partie intermédiaire (108) comprend en outre une crête, une pointe, une dent (122) ou l'une quelconque de leur combinaison.

4. Ensemble selon la revendication 3, dans lequel la partie intermédiaire (108) comprend une pluralité de crêtes concentriques (122).

5. Ensemble selon la revendication 1, dans lequel l'extrémité proximale (100) de l'ancrage osseux (99) comprend un évidement (112) ou une saillie (111) adapté(e) pour mettre en prise une caractéristique complémentaire d'un instrument d'entraînement.

6. Ensemble selon la revendication 1, dans lequel la partie non filetée (180) est sensiblement lisse.

7. Ensemble selon la revendication 1, dans lequel la pointe (106) définit trois facettes (114) ou plus.

8. Ensemble selon la revendication 1, comprenant en outre un instrument d'entraînement configuré pour appliquer une force qui sollicite la pointe (106) de l'ancrage osseux (99) dans un corps vertébral (V6, V7).
